Europäisches Patentamt

European Patent Office

Office européen des brevets

(11) Publication number: **0 521 382 A2**

# EUROPEAN PATENT APPLICATION

(21) Application number: **92110648.0**

(22) Date of filing: **24.06.92**

(51) Int. Cl.⁵: **C07C 17/156**, C07C 19/045

(30) Priority: **26.06.91 JP 154487/91**
**23.01.92 JP 10113/92**
**24.04.92 JP 106444/92**

(43) Date of publication of application:
**07.01.93 Bulletin 93/01**

(84) Designated Contracting States:
**BE DE FR GB IT NL**

(71) Applicant: **KANEGAFUCHI KAGAKU KOGYO
KABUSHIKI KAISHA
2-4 Nakanoshima 3-chome
Kita-ku Osaka-shi(JP)**

(72) Inventor: **Wachi, Shun
4-3-23 Okihama-machi, Takasago-cho
Takasago-shi, Hyogo-ken(JP)**
Inventor: **Oshima, Hiroshi
6-401, Kariguchidai 5-chome, Tarumi-ku
Kobe-shi, Hyogo-ken(JP)**
Inventor: **Terasaka, Koichi
3874-205, Nogawa, Kakatsu-ku
Kawasaki-si, Kanagawa-ken(JP)**

(74) Representative: **Türk, Dietmar, Dr. rer. nat.
Türk, Gille, Hrabal, Leifert Patentanwälte
Brucknerstrasse 20
W-4000 Düsseldorf 13(DE)**

(54) Process for preparing dichloroethane and apparatus used for the same.

(57) A process for preparing dichloroethane according to a two-stage oxychlorination reaction. The catalyst is oxidized in a reactor which is separated from a reactor where ethylene is chlorinated. The catalyst is circulated between an oxidation reactor and a chlorination reactor through a catalyst recovering line and a catalyst feed line. Preferably, an inner pressure of the oxidation reactor is higher than that of the chlorination reactor to avoid the formation of a flammable mixture.

FIG.1

The present invention relates to a process for preparing dichloroethane by oxychlorination reaction and an apparatus used for the process.

There have been known a technology in which hydrogen chloride which is by-produced in an industrial production of vinyl chloride monomer by thermal decomposition of dichloroethane is reacted with ethylene in the presence of oxygen to convert to dichloroethane again, which is called an oxychlorination process.

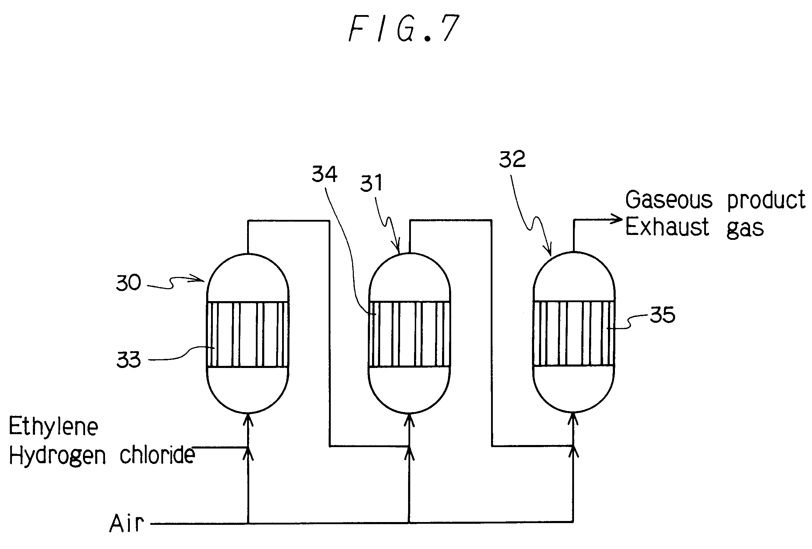

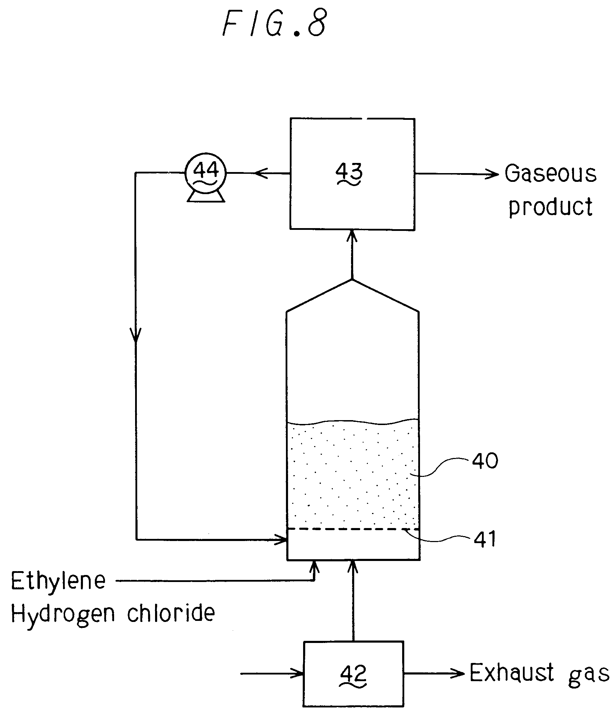

The oxychlorination process is practically carried out in an industrial scale by a method using a fixed-bed (Fig. 7) or by a method using a fluidized-bed (Fig. 8).

In the above two methods, a mixture of ethylene, a gas of oxygen source and hydrogen chloride is fed to a reactor. As the method for feeding the gas of oxygen source, there are three methods, i.e. feeding a pure oxygen gas, or feeding an oxygen enriched air, or feeding air.

When feeding the oxygen gas or the oxygen enriched air, there is a disadvantage that the oxygen gas or the oxygen enriched air must be previously prepared. But in viewpoint of exhaust gas treatment, an amount of exhaust gas is advantageously small.

On the other hand, according to the air feeding method, though air can be used as it is without any pre-treatment, a nitrogen gas exhaused in a large amount contains chlorinated organic compounds. Therefore, it is necessary to treat such a large amount of the exhaust gas before releasing it to atmosphere. In the event, the methods of feeding the oxygen gas or the oxygen enriched air are more advantageous because of a reduced amount of the exhaust gas.

In order to safely achieve the reaction, it is important for every method to make a mixing ratio of ethylene and oxygen being out of the region of flammable mixtures. For this purpose, when feeding air, an oxygen concentration makes lower by dividedly feeding the air.

When feeding oxygen or the oxygen enriched air, since such a divided feeding is not effective, the reaction is conducted out of the region of flammable mixtures by feeding an excess amount of ethylene. In this case, the excess ethylene should be separated after the reaction, compressed and returned to an inlet of the reactor for circulation. Therefore, according to the methods of feeding oxygen or the oxygen enriched air, there are problems that an excess amount of ethylene is necessary and that apparatuses for separation and compression of ethylene are necessary.

In comparison with a fixed-bed reactor of multiple tube type, when using a fluidized-bed reactor, the construction of the apparatus is simple and a catalyst powder can be packed easily. Further, an improvement of the reaction efficiency can be expected, because a uniform reaction temperature can be kept. However, there is a problem that, in the fluidized-bed reactor, it is scarcely possible that the mixing ratio goes out of the flammable mixture region, even if oxygen is dividedly fed.

As mentioned above, there are independent problems in every conventional oxychlorination process with respect to treatment of an exhaust gas, a cost for preparation of oxygen and avoidance of formation of flammable mixture. However, each of the processes has been selectively carried out depending on circumstances. Particularly, when employing the method of feeding oxygen or the oxygen enriched air for maintaining atmospheric surroundings by reducing the amount of the exhaust gas, apparatuses are newly and additionally required for the preparation of oxygen and for the avoidance of formation of flammable mixture. In the event, there are more problems in comparison with the air feeding method.

The oxychlorination process may be carried out by separate two stages, i.e. a stage for converting ethylene to dichloroethane by making ethylene contact with a catalyst and a stage for oxidizing the catalyst used for the chlorination with the gas of oxygen source. Regarding to this process, Kominami et al. reported the results by using a laboratory puls reactor with a fixed-bed (Kominami et al., Bull. Japan Petroleum Inst. 8, 27-30 (1966)). Any industrially embodied installation, however, has not been discussed until now. Namely, such a two-stage reaction is regarded as particular means for fundamental researches, and thus one could not find any industrial utilization therein.

In U.S. Pat. No. 4,668,802 is described a process in which preparation of maleic anhydride by a vapor-phase oxidation of butane in the presence of a solid catalyst is carried out by recycling and regenerating the solid catalyst, and also is described an apparatus comprising a butane oxidation reactor and a separate catalyst regeneration reactor, wherein the solid catalyst is recycled between the two reactors. However, it is silent about preparation of dichloroethane, and any practical embodiment of the apparatus is not described. Of course, there is no teaching about the problem due to use of hydrogen chloride, i.e. exhaust gas treatment.

The present inventors came to grips with industrialization of the two-stage process and found that the two-stage oxychlorination process can be accomplished by transporting and circulating a catalyst between a chlorination reactor for ethylene and an oxidation reactor for the catalyst, and then the present invention has been completed.

According to the present invention, there can be provided a process for preparing dichloroethane by using ethylene, hydrogen chloride, a gas of oxygen source and a solid catalyst, which comprises; connecting a chlorination reactor for chlorinating ethylene with an oxidation reactor for oxidizing the solid catalyst through two catalyst powder transport lines; contacting, in the chlorination reactor, the solid catalyst which is in a fluidized state with the gas of oxygen source which may contain hydrogen chloride; introducing the solid catalyst into the chlorination reactor; preparing dichloroethane by contacting, in the chlorination reactor, the solid catalyst with ethylene which may contain hydrogen chloride; removing the product effluent gases by separating the product effluent gases from the solid catalyst; and returning the solid catalyst to the oxidation reactor.

The present invention can also provide an apparatus for preparing dichloroethane by using ethylene, hydrogen chloride, a gas of oxygen source and a solid catalyst, which comprises an oxidation reactor for oxidizing the solid catalyst and a chlorination reactor for chlorinating ethylene in the presence of the solid catalyst; said oxidation reactor comprising a gas distributor for forming a fluidized-bed of the solid catalyst, a gas feed line for feeding the gas of oxygen source which may contain hydrogen chloride below the gas distributor, and a gas exhaust line for exhausting a gas which has been used for fluidizing the solid catalyst and for oxidizing the solid catalyst from the upper portion of the oxidation reactor; said chlorination reactor comprising a gaseous reactant feed line for feeding ethylene which may contain hydrogen chloride to the lower portion of the chlorination reactor, and a gaseous product effluent line for removing the product effluent gases by separating the product effluent gases from the solid catalyst; and said two reactors being connected with each other through a catalyst feed line for circulating the solid catalyst by taking out the fluidized solid catalyst from the oxidation reactor and transporting the solid catalyst to the chlorination reactor and through a catalyst recovering line for circulating the solid catalyst by transporting the solid catalyst separated in the gaseous product effluent line to the oxidation reactor.

Fig. 1 shows a diagramatic view of an embodiment of the preparation apparatus of dichloroethane according to the present invention;

Fig. 2 shows a diagramatic view of another embodiment of the apparatus according to the present invention;

Fig. 3. shows a diagramatic view of the other embodiment of the apparatus according to the present invention;

Fig. 4 shows a diagramatic view of still another embodiment of the apparatus according to the present invention;

Fig. 5 shows a graph representing the region of flammable mixtures of ethylene-oxygen-inert gas;

Fig. 6 shows a graph representing the relation between an amount of gas leakage from one reactor to the other reactor and a pressure difference of the oxidation reactor and the chlorination reactor;

Fig. 7. shows a diagramatic view of a conventional preparation apparatus of dichloroethane; and

Fig. 8 shows a diagramatic view of another conventional preparation apparatus of dichloroethane.

According to the oxyclorination process of the present invention, the two reaction stages, i.e. the stage in which ethylene is chlorinated in the presence of a catalyst and the stage in which the thus reduced catalyst is oxidized to regenerate its activity, are carried out in the separate reactors, i.e. the chlorination reactor and the oxidation reactor, while the catalyst is circulated between the two reactors. The words "oxidation" and "reduction" in this specification means change of valency of the catalyst.

In the present invention, ethylene is fed to the chlorination reactor and the gas of oxygen source is fed to the oxidation reactor. Hydrogen chloride is the essential reactant, and may be fed to at least one of the chlorination reactor and the oxidation reactor. Particularly, hydrogen chloride is preferably fed to the oxidation reactor together with the gas of oxygen source. Of course, hydrogen chloride may be fed to both reactors. According to the present invention, there are three combinations of the gaseous reactants feeding. The following explanation, however, mainly concerns with the case that hydrogen chloride is fed to the oxidation reactor.

In the present invention, since an oxidation zone where the catalyst is oxidized by using the gas of oxygen source and hydrogen chloride is provided apart from a chlorination zone where ethylene reacts in the presence of the oxidized catalyst, the exhaust gases can be released to atmosphere as they are, or even if a treatment is necessary, by such a simple treatment that a trace amount of hydrogen chloride in the exhaust gases is removed. Further, since ethylene is not intentionally admixed directly with the gas of oxygen source, even if oxygen or the oxygen enriched gas is used as the gas of oxygen source, there is less danger that the mixing ratio of ethylene and oxygen falls into the region of flammable mixtures. Therefore, the reaction can be safely conducted without using an excess amount of ethylene or without divided feeding of the gas of oxygen source. In addition, a vapor-phase oxidation (combustion) reaction between ethylene and oxygen does not substantially occur, which leads the reaction conversion of ethylene to a higher level.

Ethylene and hydrogen chloride used in the present invention are the ethylene and the hydrogen chloride used for preparation of dichloroethane by conventional oxychlorination processes, and are not particularly limited.

The gas of oxygen source used in the present invention may be air, an oxygen enriched air or oxygen, and is preferably air, because preparation of oxygen or the oxygen enriched gas is not necessary. According to the process of the present invention, the exhaust gas which predominantly contains nitrogen gas is released from the oxidation reactor, and it is not substantially required to remove any exhaust gas from the gaseous product, unreacted ethylene or unreacted hydrogen chloride. This can solve the most serious defect of the prior processes when using air. Even when oxygen gas or the oxygen enriched air is used as the gas of oxygen source, the reaction of that gas with the catalyst is conducted in the zone where ethylene is substantially absent, and the reacted gas is exhausted without contacting with ethylene, and therefore, there can be solved the defects of the prior processes when using oxygen and the like, i.e. the defect that an excess amount of ethylene is fed for avoiding formation of the flammable mixture or the defect that the excess ethylene in the gaseous product must be separated and compressed to circulate.

The comparison of the present invention with the prior industrial processes is shown in Table 1. In Table 1, X shows that there is a defect relative to other processes in the designated item.

Table 1

| Item | Fixed-bed | | Fluidized-bed | | Present invention | |
|---|---|---|---|---|---|---|
| | Oxygen method | Air method | Oxygen method | Air method | Oxygen method | Air method |
| Exhaust gas treatment | | X | | X | | |
| Oxygen feeding | X | | X | | X | |
| Compression and circulation of ethylene | X | | X | | | |
| Catalyst replacement | X | X | | | | |
| Loss of catalyst | | | X | X | X | |
| Temperature control | X | X | | | | X |

The catalyst used in the present invention is a catalyst known as the catalyst for oxychlorination, for example, a copper chloride-alumina catalyst prepared by co-precipitation or impregnation. Since the catalyst must be fluidized when contacting with the gas of oxygen source and must be circulated by being fluidized by the gaseous reactant upon the oxychlorination reaction, the catalyst preferably has an average particle size of not more than 1 mm, most preferably within the range of 20 to 300 $\mu$m. Particularly, a

catalyst of copper chloride carried on alumina having a specific surface area of 1 to 500 $m^2/g$ is preferred. When a particle size is too large, a large flow rate is required to form desired fluidized states upon oxidation by the gas of oxygen source and upon chlorination of ethylene. Consequently, there are problems that it is necessary to increase the feed rate of the gas of oxygen source in the oxidation stage, and that a contact time of ethylene with the catalyst becomes shorter in the chlorination stage. When a particle size is too small, since a separation efficiency of the catalyst from the gases becomes lower, a loss of the catalyst from the reactors becomes larger.

The preferable conditions or embodiments to achieve the process of the present invention are explained in the followings.

A feed rate of the mixed gas of the gas of oxygen source and hydrogen chloride to the catalyst oxidation reactor depends upon a particle size of the catalyst and a kind of the gas of oxygen source. When using air as the gas of oxygen source, a feed rate of the mixed gas is controlled within the range of approximately 1 to 500 cm/sec, preferably 5 to 100 cm/sec. When the feed rate is within the above range, since the catalyst forms a good fluidized state, it is easy to keep the state in the oxidation reactor for rapidly mixing the catalyst with the gases and for rapidly removing generated heat. Further an amount of the catalyst lost by splashing can be reduced. A mixing ratio of air/hydrogen chloride is preferably within the range of 1/1 to 1/0.2 (mole ratio), particularly 1/0.8 to 1/0.4.

A contact time of the mixed gas of the gas of oxygen source and hyarogen chloride with the catalyst is preferably within the range of 0.1 to 100 seconds, particularly 0.2 to 10 seconds for obtaining desired oxidation of the catalyst. A contact temperature is preferably within the range of 100° to 400°C, particularly 150° to 250°C in order to maintain the reaction activity and to inhibit vaporization of the catalyst metal to keep the structure of the catalyst. Since the oxidation reaction generates a large amount of reaction heat, it is preferable to provide a coiled tube for heat exchange in the fluidized-bed of the catalyst.

The gas of oxygen source which has been used for oxidation of the catalyst is exhausted together with unreacted hydrogen chloride. Before the exhausting, since a part of the catalyst is transported by the exhaust gas, it is preferred that the exhaust gas is released after recovering the catalyst which is separated by a solid-gas separator such as a cyclone. In case that oxidation of the solid catalyst is carried out by using only the gas of oxygen source, the exhaust gas can be released as it is, since the exhaust gas does not substantially contain other gaseous components. Even if the catalyst is returned with hydrogen chloride to the oxidation zone of the catalyst and thus the exhaust gas contains hydrogen chloride, the hydrogen chloride-containing exhaust gas can be released by a simple treatment for removing only hydrogen chloride, such as by washing with water. The separated and recovered catalyst by the solid-gas separator is returned to the oxidation zone.

The oxidized catalyst is transported from the oxidation zone to the chlorination zone where ethylene is chlorinated. The transportation method of the catalyst from the oxidation zone to the chlorination zone is not particularly limited. In view of constant transportation and prevention of leakage of the gas of oxygen source to the chlorination zone, it is preferred that the fluidized catalyst falls downward due to its deadload. An amount of the catalyst transported per hour from the oxidation zone to the chlorination zone, although it depends on the reaction efficiency in the oxidation zone, is preferably within the range of 5 to 5000 times, particularly 50 to 500 times the amount of the catalyst present in the oxidation zone. The catalyst transported to the chlorination zone contacts with ethylene to produce dichloroethane.

Though the theoretical ratio of (circulation amount of catalyst)/(feed amount of ethylene) is 2/1 as a ratio of (mole of metal carried on a catalyst)/(mole of ethylene), a larger ratio, e.g. 2/1 to 100/1 can give a high conversion in the practical operation.

Also, though the theoretical ratio of ethylene/hydrogen chloride to be used is 1/2 (mole ratio), it is preferred that one is used in excess of the other, i.e. 1/1 to 1/4, particularly 1/1.8 to 1/2.2.

The way for contacting the catalyst with ethylene is not particularly limited. For example, it is preferred that the catalyst and the gaseous reactant are fed from a lower portion of a long vertical chlorination reactor, and the chlorination reaction is carried out by conveying the catalyst on the gaseous reactant stream, since the catalyst is effectively used in the chlorination and the whole reaction zone can be easily set to preferable reaction conditions because of a uniform reaction temperature distribution.

A flow rate of those fluids in the chlorination zone of the chlorination reactor is preferably within the range of approximately 1 cm/sec to 10 m/sec, particularly 50 cm/sec to 5 m/sec, in view of fluidization and circulation of the catalyst within the apparatus. Those fluids preferably reside in the chlorination zone for 0.1 to 100 seconds, particularly 0.2 to 10 seconds. As a temperature of the chlorination zone there can be generally employed the range of 100°C to 400°C, preferably 150 to 250°C.

After the completion of the reaction, the solid catalyst is separated from the gaseous product containing the gaseous reactant, and is returned to the oxidation zone, then circulated. The product effluent gas is introduced to an after-treatment stage, and there the desired dichloroethane is separated from ethylene.

According to the above-mentioned process, dichloroethane can be produced in a reaction conversion of 50 to 100 % on the basis of ethylene.

The above explanation relates to the case that the oxidation of the catalyst is carried out by using a mixed gas of the gas of oxygen source and hydrogen chloride. In case that the catalyst is oxidized by using only the gas of oxygen source which is air, a feed rate is preferably within the range of 1 to 500 cm/sec, particularly 5 to 100 cm/sec.

When the catalyst which is fed to the chlorination zone contains chloride atom in a sufficient amount for the chlorination (the manner in which the chloride atom is contained is not clear), only ethylene may be fed to the chlorination zone. When containing an insufficient amount of chloride atom, hydrogen chloride may be additionally fed to the chlorination zone.

As is explained above, in the process of the invention, careful attention is paid to prevent contact and mixing of ethylene with the gas of oxygen source for avoiding the danger of explosion.

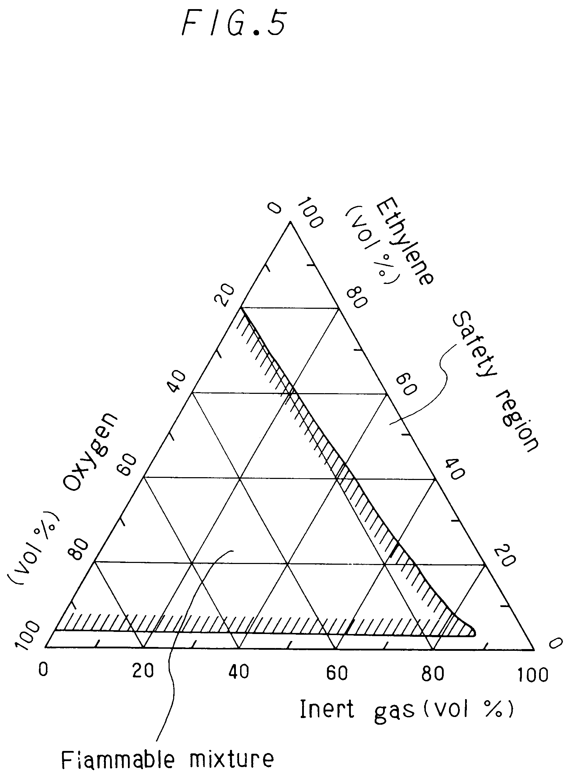

The flammable mixture region which is formed by ethylene, oxygen and an inert gas is shown in Fig. 5. Since the lower limit of the flammable mixture region is about 2 % of ethylene content and a safety region is narrow, the practical operation should be carried out within the wider safety region which is formed above the upper limit.

Even in this two-stage oxychlorination process, since the catalyst is transported, mixing of the gases through the catalyst transport lines may happen. As a result, the gas which has been used for oxidizing the catalyst may be contaminated with the unreacted ethylene or the dichloroethane product, or the gas of oxygen source may leak into the chlorination reactor.

In the above situations, in case that a small amount of oxygen leaks out to the chlorination reactor and is mixed with ethylene, the safety operation region is wide because the mixture is near the upper limit of the flammable mixture region shown in Fig. 5. In case that ethylene leaks out to the oxidation reactor and is mixed with the gas containing oxygen, however, there is a danger that the leakage of a relatively small amount of ethylene forms a flammable mixture becasue the mixture is near the lower limit of the region. Further, when the exhaust gas from the oxidation reactor mainly containing nitrogen gas and water which is produced by the oxidation are contaminated with chlorinated hydrocarbons leaked out from the chlorination reactor, the exhaust gases must be subjected to a cleaning treatment prior to the release to surroundings. Therefore, among the two cases, the case that the gas leaks out to the oxidation reactor from the chlorination reactor has more problems.

In order to effectively inhibit such a gas transfer through the catalyst transport lines, particularly the gas transfer from the chlorination reactor to the oxidation reactor, an inner pressure of the oxidation reactor is maintained higher than an inner pressure of the chlorination reactor. According to this method, it is possible to prevent ethylene from leaking out to the oxidation reactor and to prevent the gas mixture from going beyond the lower limit of the flammable mixture region shown in Fig. 5. On the other hand, according to the method, though an amount of oxygen which leaks out to the chlorination reactor is increased, there is no substantial problem, because the formed gas mixture is within the relatively wide safety region above the upper limit.

A pressure difference between the two reactors is preferably within the range of 0.01 to 10 $mH_2O$, particularly 0.05 to 1 $mH_2O$. When the pressure difference is lower than 0.01 $mH_2O$, the effect obtained by setting the pressure difference is not remarkable. When higher than 10 $mH_2O$, almost of the gas in the oxidation reactor flows into the chlorination reactor, and further controlling of the catalyst transportation becomes difficult. The catalyst can move from the chlorination reactor to the oxidation reactor by gravity against the pressure difference.

In order to more effectively prevent the gases from leaking through the catalyst transport lines, an inert gas may be introduced into the two catalyst transport lines between the two reactors. This introduction can inhibit the mixing of ethylene with air or can prevent the exhaust gas such as nitrogen gas from mixing with ethylene or dichloroethane, more effectively. Examples of the inert gas are, for instance, nitrogen gas or the like. The inert gas is preferably fed in such a minimum amount that the catalyst powder layer can be fluidized. A suitable flow rate, which varies with a form of the catalyst particle, is generally set so that a superficial velocity on the basis of a sectional area of the catalyst feed line pipe falls into the range of 0.005 to 0.5 m/sec.

The present invention also relates to an apparatus used for preparing dichloroethane by the two-stage oxychlorination process.

The apparatus of the invention is explained hereinbelow by referring the accompanying drawings. For simplifying the explanation, the following explanation mainly directs to a case that the gas fed to the oxidation reactor is a gas mixture of air and hydrogen chloride (hereinafter referred to as "oxidation gas") and that to the chlorination reactor is fed only ethylene. Of course, the apparatus of the present invention may also be employed in the other cases or their combinations.

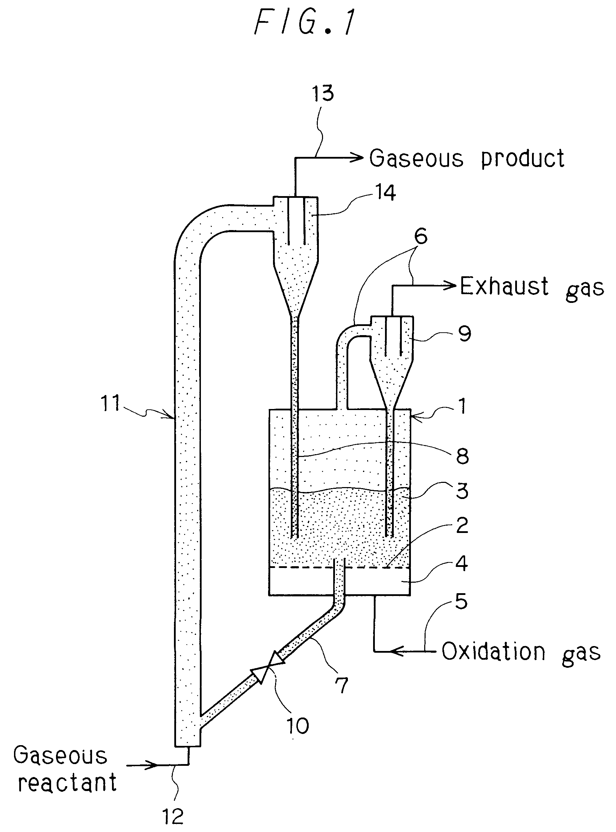

In Fig. 1, the numeral 1 designates an oxidation reactor for oxidizing a solid catalyst 3 which is in a fluidized state above a gas distributor 2. The oxidation reactor 1 has a gas feed line 5 for feeding the oxidation gas to a windbox 4, a gas exhaust line 6 for exhausting the oxidation gas after contacting with the solid catalyst 3, a catalyst feed line 7 for transporting the catalyst 3 to a chlorination reactor 11 for a chlorination reaction, and a catalyst recovering line 8 for recovering the catalyst which has been used in the chlorination reaction to the oxidation reactor 1.

The oxidation reactor 1 is preferably a vertical tower-like reactor having a space above the fluidized-bed of the catalyst, because the catalyst powder is prevented from splashing. The gas distributor 2 is preferably provided near the bottom portion of the oxidation reactor, because the catalyst powder can be wholly fluidized in a good state.

As the gas distributor 2, there may be employed a porous plate which is capped for avoiding plugging of pores, or a nozzle. In a relatively small apparatus, there may be preferably employed, for example, a sintered metal plate in view of simplification.

The catalyst feed line 7 is preferably provided so that the line extends downward with inclination from the oxidation reactor 1 to the lower portion of the chlorination reactor 11. With the catalyst feed line 7 is preferably provided, for example, a control valve 10 for the catalyst powder flow such as a butterfly valve, a ball valve or a throttle valve.

The gas exhaust line 6 is preferably provided with a cyclone 9 in order to separate the catalyst powder conveyed on the exhaust gas from the exhaust gas, then to recover and return it to the oxidation reactor 1.

With the chlorination reactor 11 are connected the catalyst feed line 7 for transporting the catalyst from the oxidation reactor 1, a gaseous reactant feed line 12 for feeding ethylene as the gaseous reactant, and a cyclone 14 having the catalyst recovering line 8 for returning the catalyst to the oxidation reactor after the chlorination and a gaseous product effluent line 13 for taking out the gaseous product.

The chlorination reactor is preferably a vertical tower-like reactor.

With respect to the relative position of the chlorination reactor 11 and the oxidation reactor 1, it is preferred, as shown in Fig. 1, that the oxidation reactor 1 is positioned between the levels of the top and the bottom of the chlorination reactor 11.

The catalyst feed line 7 is preferably connected with the lower portion of the chlorination reactor 11. The gaseous reactant feed line 12 is preferably connected with the chlorination reactor 11 at a point of the bottom portion of the reactor below the point where the catalyst feed line 7 is connected. According to this relationship, the chlorination reaction can be suitably carried out by conveying the catalyst with the gaseous reactant.

The cyclone 14 having the catalyst recovering line 8 and the gaseous product effluent lines 13 for taking out the gaseous product is preferably provided at the upper portion, particularly on the top of the chlorination reactor 11, becasue the separation of the catalyst from the gaseous product can be smoothly, efficiently carried out.

The catalyst recovering line 8 preferably communicates the bottom portion of the cyclone 14 with the fluidized portion of the catalyst 3 in the oxydation reactor 1.

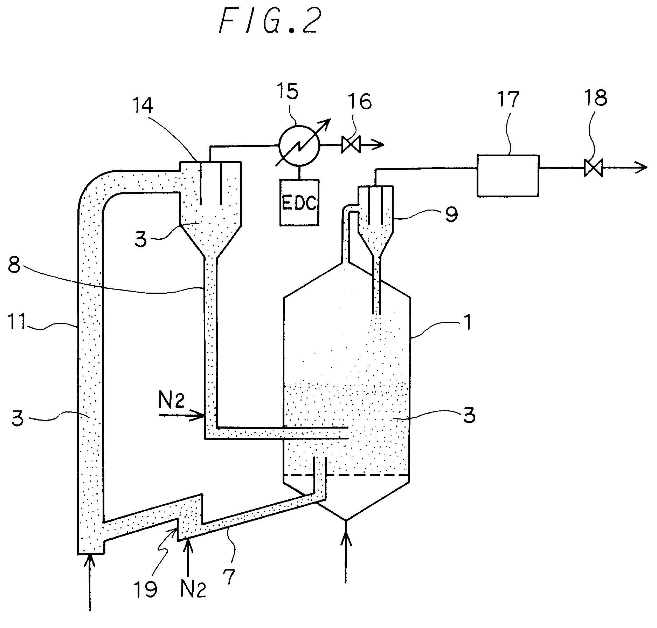

Another embodiment is shown in Fig. 2.

In Fig. 2, the chlorination reactor 11 for chlorinating ethylene and the oxidation reactor 1 for oxidizing the solid catalyst after the chlorination are connected with each other through the catalyst recovering line 8 and the catalyst feed line 7.

Ethylene is chlorinated by feeding ethylene to the lower portion of the chlorination reactor 11, and contacting ethylene with the solid catalyst 3 which is fluidized by ethylene. The thus reacted solid catalyst is exhausted together with the dichloroethane product from the upper portion of the chlorination reactor 11, and then separated from the gaseous product by the cyclone 14. Dichloroethane (EDC) in the gaseous product is condensed and recovered by a condenser 15, and the remaining non-condensed gases containing unreacted ethylene is exhausted through an exhaust valve 16. The separated solid catalyst is transported to the oxidation reactor 1 through the catalyst recovering line 8. To the lower portion of the oxidation reactor 1, the oxidation gas is fed. The solid catalyst is fluidized and oxidized by the oxidation gas. The solid catalyst 3 in the oxidation reactor 1 is transported to the lower portion of the chlorination reactor 11 through the catalyst feed line 7. The gases (steam and nitrogen gas) after the oxidation reaction are separated from the catalyst, washed with water in exhaust gas cleaning facilities 17 to remove unreacted hydrogen chloride, and then released into atmosphere through an exhaust valve 18.

In this embodiment, the inner pressure of the oxidation reactor 1 is regulated to be higher than the inner pressure of the chlorination reactor 11. This pressure difference can be easily obtained by opening or closing the exhaust valve 16 or 18, or by passing the exhaust gas through a controllable water head. The pressure difference may be controlled according to the values detected by pressure sensors (not shown in Fig. 2) provided with the reactors.

Further, an inert gas such as nitrogen gas is fed to the catalyst feed line 7 and/or the catalyst recovering line 8. When the inert gas is fed to the catalyst feed line 7, it is possible not only to prevent mixing of the gases in both reactors, but also to control the amount of the catalyst transported to the chlorination reactor 11 by regulating the flow rate of the inert gas. Accordingly, the control valve for the catalyst powder flow shown in Fig. 1 is not necessary. When feeding the inert gas to the catalyst recovering line 8, it is possible to prevent the leakage of ethylene to the oxidation reactor 1 which should be most carefully avoided.

The safety operation can be more insured by providing a Z-pipe portion 19 on the way of the catalyst feed line 7. If the amount of the catalyst 3 in the oxidation reactor 1 is decreased so that the upper level of the fluidized-bed goes down below the level of the inlet of the catalyst feed line 7, the oxidation gas comes into the catalyst feed line 7. In such a case, when the Z-pipe portion 19 is provided, since the catalyst is accumulated densely in the portion and the portion is not kept empty, the leakage of the gas can be prevented.

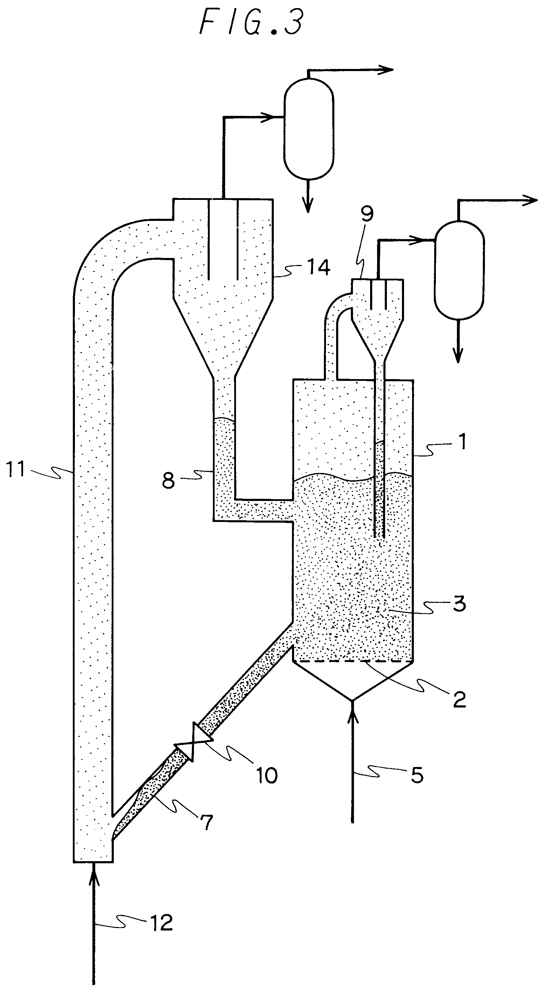

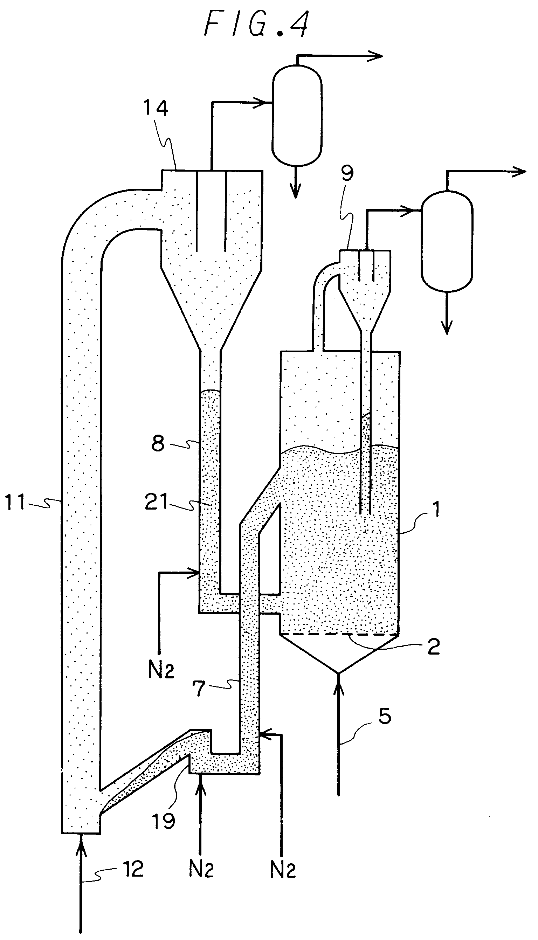

In Fig. 3 and Fig. 4, there can be shown embodiments which are characterized by the catalyst feed line 7 and the catalyst recovering line 8. In the drawings the same parts as in Fig. 1 are designated by the same symbols.

According to the embodiment of Fig. 3, the catalyst is taken out to the catalyst feed line 7 from the oxidation reactor 1 at the bottom portion which is positioned above the gas distributor 2. The catalyst recovering line 8 communicates with the upper portion of the fluidized-bed in the oxidation reactor 1. According to the arrangement, there is an advantage that a sufficient residence time of the catalyst can be obtained while the catalyst powder moves from the upper portion to the lower portion.

In the embodiment of Fig. 4, the catalyst recovering line 8 is connected with the oxidation reactor 1 at its bottom portion which is positioned above the gas distributor 2. The inlet of the catalyst feed line 7 is provided at the upper portion of the fluidized-bed. This embodiment is particularly preferable in view of circulation of the catalyst and prevention of gas mixing.

The function of the catalyst feed line and the catalyst recovering line is to transport the catalyst powder smoothly. In addition, it is desired that leakage and mixing of the gases through the transportion is inhibited as low as possible. For obtaining the effect, the catalyst recovering line 8 is preferably introduced into the bottom portion of the fluidized-bed. Also, the catalyst feed line 7 is connected at a point near the upper portion of the fluidized-bed in the oxidation reactor 1, and the catalyst is fed to the bottom portion of the chlorination reactor 11.

The aim of the introduction of the catalyst recovering line 8 into the bottom portion of the fluidized-bed in the oxidation reactor 1 is to extend the catalyst powder layer 21 in the line 8 so as to reduce leakage of the gas. The fluidized-bed in the oxidation reactor 1 is positioned below the cyclone 14 which is provided at the upper portion of the chlorination reactor 11, thereby the catalyst can move into the oxidation reactor 1 by its deadload through the catalyst recovering line 8. On the other hand, since the flow of gases is mainly governed by the pressure difference, the gas in the oxidation reactor flows from the oxidation reactor of a higher pressure in the direction of the cyclone 14 of a relatively lower pressure. At that time, the extended powder layer 21 in the line 8 can prevent the gas flow.

The catalyst is taken out by the catalyst feed line 7 preferably from the upper portion of the fluidized-bed in the oxidation reactor 1, because oxidation gas leakage to the chlorination reactor 11 can be reduced due to a lower pressure of the upper portion. On the other hand, the flow rate of the gaseous reactant to the chlorination reactor 11 is controlled to a superficial velocity within the range of 1 cm/sec to 20 m/sec, preferably 10 cm/sec to 5 m/sec, particularly faster than 0.5 m/sec. Since the density of the fluidized-bed in the chlorination reactor 11 becomes small at that flow rate, a good fluidized state in which a pressure loss in the direction of hight is small can be obtained. According to the conditions, since the bottom portion of the chlorination reactor 11 can also be maintained at a pressure lower than the pressure in the oxidation reactor, the above flow rate may preferably be employed in view of controlling the catalyst transportation and the gas flow.

In the embodiment of Fig. 4, there is provided the Z-pipe portion 19, as is the same in Fig. 2. The inert gas is fed to the catalyst recovering line 8 at one point, and to the catalyst feed line 7 at two points. The inert gas fed to the Z-pipe portion 19 is to fluidize and transport the catalyst, and the inert gas fed to the point near the oxidation reactor is to make the movement of the catalyst powder in the line smooth.

According to the present invention, any flammable mixture cannot be formed, since oxidation of the catalyst and chlorination of ethylene are carried out in the different zones, and preferably the inner pressure of the oxidation reactor is higher than the inner pressure of the chlorination reactor. As a result, it is not necessary to provide a plurality of reactors for dividing the gas of oxygen source as in the prior processes. Also, it is not necessary to feed an excess of ethylene and circulate the excess, unreacted ethylene after separation and condensation.

Further, since the gas of oxygen source is not fed directly to the chlorination reactor, a consumption of ethylene due to the vapor-phase oxidation (combustion) reaction can be reduced.

When using air as the gas of oxygen source, a process for previously preparing oxygen is not necessary. Further, since the exhaust air (mainly containing nitrogen gas) is exhausted separatively from the gaseous product, air polution by chlorinated organic compounds can be substantially avoided, and then the exhaust air can be released to atmosphere after a very simple treatment.

The present invention is more specifically described and explained by means of the following Examples. It is to be understood that the present invention is not limited to the Examples, and various changes and modifications may be made in the invention without departing from the spirit and scope thereof.

EXAMPLE 1

For this example, the following apparatus as shown in Fig. 1 was used. The oxidation reactor 1 is a vertical cylindrical reactor having an inner diameter of 10 cm and a hight of 1.5 m. With the reactor is provided the gas distributor 2 of a sintered metal plate at the bottom portion. The reactor has the gas feed line 5 at the bottom portion for feeding air to the windbox 4, and the gas exhaust line 6 having the cyclone 9 at the upper portion. The catalyst feed line 7 having a diameter of 2 cm is provided so as to extend from the bottom of the oxidation reactor 1 through the center of the gas distributor 2 to the point above the gas distributor 2 by 5 cm, and then opens in the fluidized-bed of the catalyst. The catalyst feed line 7 has the control valve 10 for the catalyst powder flow, and is connected to the lower portion of the chlorination reactor 11. Also, the catalyst recovering line 8 having a diameter of 2 cm extends from the cyclone 14 to the fluidized-bed in the oxidation reactor 1. The chlorination reactor 11 is a straight tube having an inner diameter of 4 cm and a hight of 5 m. At the upper portion of the reactor 11, the cyclone 14 is provided for separating the solid catalyst. The catalyst recovering line 8 is connected to the lower portion of the cyclone, and the gaseous product effluent line 13 is connected to the upper portion of the cyclone. To the lower portion of the chlorniation reactor 11 are connected the catalyst feed line 7 and the gaseous reactant feed line 12.

The oxidation reactor 1 was charged with 7 kg of the oxychlorination catalyst having an average particle size of 100 $\mu$m which was prepared by carrying copper chloride on alumina powder. The catalyst powder was fluidized at 220°C by feeding air to the windbox 4 at the rate of 130 $\ell$/min. The exhaust gas was exhausted through the gas exhaust line 6. The catalyst was transported to the chlorination reactor 11 through the catalyst feed line 7 at the rate of about 700 g/min by regulating the control vlave 10. At the same time, ethylene and hydrogen chloride were fed through the gaseous reactant feed line 12 at the rates of 50 $\ell$/min and 100 $\ell$/min, respectively. The reaction temperature in the chlorination reactor 11 was 230°C.

The gaseous product and the catalyst were separated from each other by the cyclone 9, and the catalyst was returned to the oxidation reactor 1. The gaseous product was taken out by the gaseous product effluent line 13 to obtain dichloroethane.

The exhaust gas could be released without any treatment, because the oxidation of the catalyst and the chlorination of ethylene were carried out in the separate zones. Also, since ethylene was not directly mixed with the gas of oxygen source, any flammable mixture was not formed and the reaction could be completely carried out. Further, the treatment of the gaseous product was easy.

COMPARATIVE EXAMPLE 1

Dichloroethane was prepared according to the fixed-bed oxychlorination process by using air and the apparatus shown in Fig. 7.

In Fig. 7, the numerals 30, 31 and 32 represent conventional reactors of multiple tube type with catalyst packed beds 33, 34 and 35 for removing the heat of reaction. The tubes having an inner diameter of 3 cm and a length of 2 m which are installed in each reactor are charged with the catalyst which is the same as in EXAMPLE 1.

Ethylene and hydrogen chloride were fed to the lower portion of the reactor 30 and were taken out from the upper portion, then fed to the lower portion of the reactor 31, taken out from the upper portion thereof, subsequently fed to the lower portion of the reactor 32, and finally taken out from the upper portion thereof.

In this operation, in order to avoid formation of a flammable mixture, air was dividely fed so that one-third of the required air was fed to the lower portion of the reactor 30, one-third was fed to the lower portion of the reactor 31, and the remaining one-third was fed to the lower portion of the reacter 32.

During the reaction, the temperature of the tube of each reactor varied in the direction of length. Namely, the temperature was distributed from $200°C$ to $300°C$, and was highest at the center of the tube.

The gaseous product from the upper portion of the reactor 32 comprised nitrogen (about 50 %), dichloroethane (25 %) and steam (25 %). For condensation and separation of dichloroethane from the gaseous product, a condenser having a large capacity was required. Further, since the exhaust gas after the separation contained chlorinated organic compounds, certain cleaning facilities were necessary for releasing the exhaust gas to atmosphere.

COMPARATIVE EXAMPLE 2

Dichloroethane was prepared according to the conventional fluidized-bed oxychlorination process by using oxygen and the apparatus shown in Fig. 8.

The numeral 40 in Fig. 8 represents a fluidized-bed type reactor having a gas distributor 41 which is the same as the gas distributor in Fig. 1. The reactor 40 was charged with the catalyst which is the same as in EXAMPLE 1, and thereto were fed ethylene, hydrogen chloride and oxygen which was prepared by an oxygen preparation process 42. After the reaction was carried out at $230°C$, the gaseous product was taken out from the upper portion of the reactor 40. The gaseous product was fed to a separation process 43 to separate an excess ethylene, and then dichloroethane was obtained. The separated ethylene was compressed by a compressor 44, and returned to the reactor 40. In order to avoid formation of a flammable mixture, ethylene was circulated so that an amount of ethylene at the inlet of the reactor 40 is four times larger than a feed amount of oxygen.

A condenser having a large capacity was required for condensation and separation of dichloroethane as in COMPARATIVE EXAMPLE 2. Further, the compressor for ethylene was necessary.

EXAMPLE 2

The following experiment was conducted in order to show the relation of a pressure difference between the chlorination reactor and the oxidation reactor to an amount of gas leakage.

As an apparatus for the experiment, the apparatus of Fig. 2 was employed, and air was fed to each reactor instead of the gaseous reactant. When determining an amount of gas leakage from the oxidation reactor 1 to the chlorination reactor 11, Freon-12 was mixed as a tracer into the air which was fed to the oxidation reactor 1, and a concentration of Freon in the exhaust gas from the cyclone 14 was measured. On the other hand, when determining an amount of gas leakage from the chlorination reactor 11 to the oxidation reactor 1, Freon was mixed into the air which was fed to the chlorination reactor, and a concentration of Freon in the exhaust gas from the cyclone 9 was measured.

A pressure difference was produced between the two reactors by regulating the exhaust valves 16, 18, and the produced pressure difference $\Delta P$ (inner pressure of the oxidation reactor - inner pressure of the chlorination reactor) was increased gradually from -200 mmH$_2$O.

As the chlorination reactor 11, a pipe having a diameter of 3 cm and a hight of 2 m was used, and as the oxidation reactor 1, a cylindrical reactor having a diameter of 10 cm and a hight of 1 m which was charged with 2 kg of alumina powder having an average particle size of about 100 $\mu$m was used. To the chlorination reactor and the oxidation reactor was fed air at the flow rates of 1.5 $\ell$/sec and 1 $\ell$/sec, respectively. The flow rate of the tracer gas, i.e. Freon was 0.5 $\ell$/min. No inert gas was introduced to the catalyst recovering line 8 and the catalyst feed line 7.

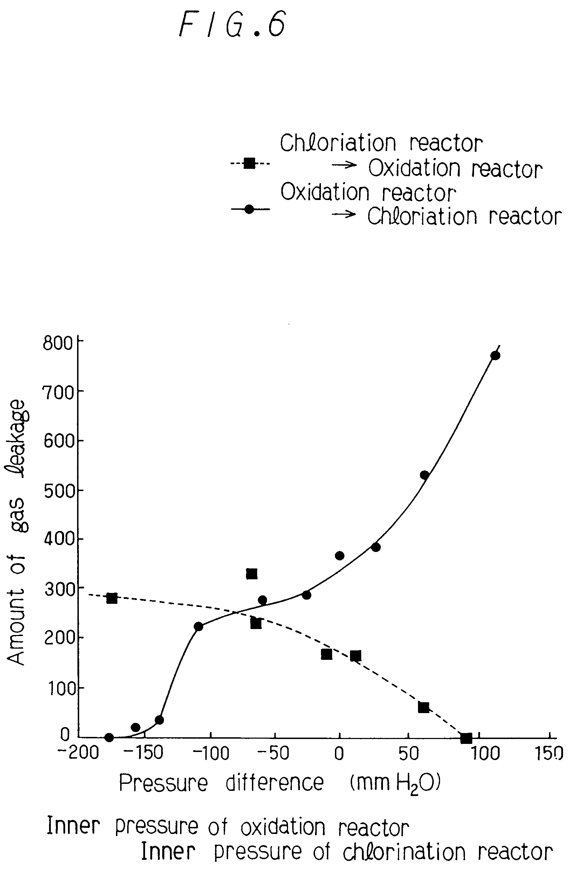

The results are shown in Fig. 6.

As is clear from Fig. 6, when the inner pressure of the oxidation reactor becomes high, it is possible to inhibit the leakage of ethylene and dichloroethane from the chlorination reactor to the oxidation reactor which is the most dangerous and requires an expensive cleaning treatment for the exhaust gas.

EXAMPLE 3

Dichloroethane was prepared by using an apparatus shown in Fig. 2. As the chlorination reactor 11, a pipe having a diameter of 3 cm and a hight of 3 m was used, and as the oxidation reactor 1, a cylindrical reactor having a diameter of 10 cm and a hight of 1.5 m was used. The other conditions were as follows.

| Catalyst: | 3 kg of alumina powder having an average particle size of about 100 $\mu$m (specific surface area of 200 m$^2$/g) and carrying thereon copper chloride in amount of about 10 % by weight. |
| Chlorination reaction: | Feeding ethylene at the rate of 40 $\ell$/min, and the reaction was carried out at 210°C. |
| Oxidation reaction: | Feeding hydrogen chloride at the rate of 80 $\ell$/min and air at the rate of 105 $\ell$/min, and the reaction was carried out at 210°C. |
| Pressure difference ($\Delta$ P): | The pressure of the oxidation reactor is higher by about 20 cmH$_2$O. |
| Inert gas feeding to the lines: | Feeding at the rate of 0.5 $\ell$/min to the catalyst recovering line 8 and to the catalyst feed line 7. |
| Cleaning treatment: | Washing with 10 % aqueous solution of NaOH. |

The two-stage oxychlornation reaction was conducted under those conditions to obtain dichloroethane in a conversion of about 80 % based on ethylene. The exhaust gas from the cleaning facilities comprised almost nitrogen and oxygen, and did not contain any chlorinated hydrocarbon or ethylene which must be burned.

**Claims**

1. A process for preparing dichloroethane by using ethylene, hydrogen chloride, a gas of oxygen source and a solid catalyst, which comprises; connecting a chlorination reactor for chlorinating ethylene with an oxidation reactor for oxidizing the solid catalyst through two catalyst powder transport lines; contacting, in the chlorination reactor, the solid catalyst which is in a fluidized state with the gas of oxygen source which may contain hydrogen chloride; introducing the solid catalyst into the chlorination reactor; preparing dichloroethane by contacting, in the chlorination reactor, the solid catalyst with ethylene which may contain hydrogen chloride; removing product effluent gases by separating the product effluent gases from the solid catalyst; and returning the solid catalyst to the oxidation reactor.

2. The process of Claim 1, wherein the gas of oxygen source which contains hydrogen chloride is fed to the oxidation reactor.

3. The process of Claim 1, wherein an inner pressure of the oxidation reactor is maintained higher than an inner pressure of the chlorination reactor.

4. The process of Claim 3, wherein an inner pressure difference of the oxidation reactor and the chlorination reactor is maintained within the range of 0.01 to 10 mH$_2$O.

5. The process of Claim 3, wherein an inert gas is introduced into at least one of the two lines which connect the oxidation reactor with the chlorination reactor.

6. An apparatus for preparing dichloroethane by using ethylene, hydrogen chloride, a gas of oxygen source and a solid catalyst, which comprises an oxidation reactor for oxidizing the solid catalyst and a chlorination reactor for chlorinating ethylene in the presence of the solid catalyst; said oxidation reactor comprising a gas distributor for forming a fluidized-bed of the solid catalyst, a gas feed line for feeding the gas of oxygen source which may contain hydrogen chloride below the gas distributor, and a gas exhaust line for exhausting a gas which has been used for fluidizing the solid catalyst and for oxidizing the solid catalyst from the upper portion of the oxidation reactor; said chlorination reactor comprising a gaseous reactant feed line for feeding ethylene which may contain hydrogen chloride to the lower portion of the chlorination reactor, and a gaseous product effluent line for removing product effluent gases by separating the product effluent gases from the solid catalyst; and said two reactors being connected with each other through a catalyst feed line for circulating the solid catalyst by taking out the fluidized solid catalyst from the oxidation reactor and transporting the solid catalyst to the chlorination reactor and through a catalyst recovering line for circulating the solid catalyst by transporting the solid catalyst separated in the gaseous product effluent line to the oxidation reactor.

7. The apparatus of Claim 6, wherein the catalyst recovering line communicates with the fluidized-bed of the solid catalyst in the oxidation reactor at the lower portion of the fluidized-bed, and the catalyst feed line communicates with the fluidized-bed of the solid catalyst in the oxidation reactor at a point above the point where the catalyst recovering line communicates witht he fluidized-bed.

# FIG.1

→ Gaseous product

→ Exhaust gas

← Oxidation gas

Gaseous reactant →

# FIG.2

EP 0 521 382 A2

# FIG.3

# FIG.4

# F I G. 5

# F I G . 6

Chloriation reactor
--■-- → Oxidation reactor
Oxidation reactor
● → Chloriation reactor

Inner pressure of oxidation reactor
Inner pressure of chlorination reactor

# FIG.7

Ethylene
Hydrogen chloride

Air

Gaseous product
Exhaust gas

30  33  34  31  32  35

EP 0 521 382 A2

# FIG.8

Gaseous product

Ethylene
Hydrogen chloride

Exhaust gas